Europäisches Patentamt

European Patent Office

Office européen des brevets

(19)

(11) Publication number : **0 327 194 B1**

(12) **EUROPEAN PATENT SPECIFICATION**

(45) Date of publication of patent specification :
08.07.92 Bulletin 92/28

(51) Int. Cl.[5] : **C07C 381/12**

(21) Application number : **89300075.2**

(22) Date of filing : **05.01.89**

(54) **Synthesis of triarylsulphonium salts.**

(30) Priority : **05.02.88 US 152729**

(43) Date of publication of application :
**09.08.89 Bulletin 89/32**

(45) Publication of the grant of the patent :
**08.07.92 Bulletin 92/28**

(84) Designated Contracting States :
**DE FR GB**

(56) References cited :
RESEARCH DISCLOSURE, no. 290, June 1988,
page 414, no. 29082, New York, NY, US;
"Synthesis of triarylsulfonium salts"
JOURNAL OF THE AMERICAN CHEMICAL
SOCIETY, vol. 73, May 1951, pages 1965-1967,
Easton, US; B.S. WILDI et al.: "The prep-
aration of triarylsulfonium halides by the ac-
tion of aryl grignard reagents on diphenyl
sulfoxide"

(56) References cited :
JOURNAL OF THE AMERICAN CHEMICAL
SOCIETY, vol. 93, no. 23, 17th November 1971,
pages 6077-6086, Easton, US; R.W.
LaROCHELLE et al.: "Reactions of
organolithiums with arylsulfonium salts"

(73) Proprietor : **International Business Machines
Corporation
Old Orchard Road
Armonk, N.Y. 10504 (US)**

(72) Inventor : **Dektar, John Louis
5583 Walnut Blossom Dr., Apt. 20
San Jose, CA 95123 (US)**
Inventor : **Hacker, Nigel Patrick
18017 Hillwood Lane
Morgan Hill, CA 95037 (US)**

(74) Representative : **Burt, Roger James, Dr.
IBM United Kingdom Limited Intellectual
Property Department Hursley Park
Winchester Hampshire SO21 2JN (GB)**

EP 0 327 194 B1

Jouve, 18, rue Saint-Denis, 75001 PARIS

## Description

The present invention is concerned with an improved synthesis of triarylsulphonium salts.

Triarylsulphonium salts are used as photo-acid initiators for polymerization and ester cleavage. They are also used as radical photoinitiators. They have, however, suffered from the disadvantage from being extremely expensive and also needing to be exceptionally pure when they are used.

The most commonly used synthesis of triarylsulphonium salts is that given by Crivello and Lam, J. Polym. Sci., 17, 977 (1979). This method is usually called the "iodonium salt route". The method has the disadvantage of requiring the use of toxic iodonium salts. It has the additional disadvantage in that it is a two-step process and that expensive reagents are used in the lower yielding first step of the two-step process. The products from each step are impure, being isolated as colored oils.

Multiple recrystallizations are required in order to obtain white crystalline products.

The literature also describes another method for the synthesis of triphenylsulphonium salts. This two-step process is described by the following equations:

STEP 1

$$n \ ArMgX + (Ar)_2SO \longrightarrow (Ar)_3SOMgX \ \underset{\longrightarrow}{\underline{2HX}}$$

$$(Ar)_3S^+ \ X + MgX_2 + H_2O$$

STEP 2

$(Ar)_3S^+ X + ZMF_6 \rightarrow (Ar)_3S^+ MF^-_6 + Z$

wherein Ar is aromatic such as phenyl, tolyl, X is a halogen such as bromide, and Z is a alkali metal such as sodium and M is antimony, arsenic or phosphorus. Step 1 of this synthesis is described in Wildi et al, J. Amer. Chem. Soc., 73, 1965 (1951) and LaRochelle et al, J. Amer. Chem. Soc., 93, 6077 (1971). Step 2 of this synthesis is described by Smith, U. S. Patent 4,173,476.

The above-described process is greatly improved by certain changes in the procedure. In the prior art, the Grignard reaction used either ether or ether-benzene co-solvents. According to the present invention, greatly higher yields are obtained using a solvent which is a mixture of aromatic and aliphatic hydrocarbons. Using the solvent mixture of the present invention also has the advantages of shorter reaction times, 3 hours versus 18 hours in the prior art. Furthermore, less Grignard reagent can be used. The present invention requires only 3 equivalents of Grignard reagent to give optimum yield of product, whereas the prior art requires 5 equivalents of Grignard reagent and gives less product (Table I). In the prior art, the second step of the reaction was carried out using aqueous solvents. We have now found that the procedure is greatly improved when a non-aqueous solvent is used. Furthermore, we have found that the throughput for the process is improved when a metal-like salt such as for example ammonium salt is used for the metathetical second step of the process. Using the improved process of the present invention, the products of each of the two steps of the reactions are white crystals. The process also has the additional advantage in that, in the process of the present invention, the expensive MX_6 anion is used in the higher yielding second step.

Table I below lists synthesis results obtained by the process of the present invention and compares them with those obtained in the prior art, specifically, the Wildi et al reference which is labelled 1, and the LaRochelle et al reference, labelled 2, as mentioned above.

TABLE I.   YIELDS FOR GRIGNARD REACTION

| SOLVENT(S) | EQUIV. PhMgBr | TIME hr. | %YIELD This work[a] | Ref. 1[b] | Ref. 2[b] |
|---|---|---|---|---|---|
| tetrahydrofuran | 2 | 16 | 0 | -- | -- |
| benzene | 2 | 12 | -- | 14 | -- |
| 1:1 heptane-benzene | 2 | 1 | 39 | -- | -- |
| 1:1 heptane-benzene | 2 | 18 | 47 | -- | -- |
| 2:1 heptane-toluene | 2.5 | 3 | 44 | -- | -- |
| 1:1 heptane-benzene | 3 | 3 | 55 | -- | -- |
| 3:1 heptane-benzene | 3 | 18 | 56 | -- | -- |
| benzene | 5 | 24 | 27-45 | 49 | 39 |

a. All yields are for pure crystallized material. Add ca. 5% for crude yield.
b. All yields are for crude product.
The following Examples illustrate the present invention.

Examples

Grignard Reaction.

The Grignard reagent, (Aldrich) or prepared from stoichiometric amounts of bromoarene and Mg, was heated under vacuum to remove solvent. This produced a viscous semi-crystalline mass. To this was added benzene or toluene (about 2 ml / mmol of Grignard reagent). n-Heptane was then added to give the desired ratio of solvents. The solution was heated to about 80°C and a nearly saturated solution of diarylsulphoxide in benzene or toluene was added. Heating was continued for time indicated in Table I. The cooled reaction mixture was quenched with 3 equivalents of 25% aqueous HBr per equivalent of Grignard reagent (exotherm!). Two layers separated and the organic layer was extracted with 5% aqueous HBr. The combined aqueous solutions were extracted with chloroform. The combined organic extracts were dried over $MgSO_4$, filtered and evaporated. The residue was dissolved in dichloromethane and the product precipitated by adding ether.

Metathesis Reactions.

A. 1.72g of triphenylsulphonium bromide (prepared above) and .82g of ammonium hexafluorophosphate were mixed in 60 ml of acetonitrile and stirred for 15 hrs. The suspension was filtered and the filtrate evaporated to yield a white solid in quantitative yield. Recrystallization from anhydrous ethanol gave white needles m.p. 203-5°C (86% recovery).
B. 1.72g of triphenylsulphonium bromide (prepared above) and 1.38g of potassium hexafluoroantimonate were mixed in 60 ml of acetone and stirred for 15 hrs. The suspension was filtered and the filtrate evaporated to yield a white solid in quantitative yield. Recrystallization from anhydrous ethanol gave white needles m.p. 178-9°C (89-95% recovery).
C. 2.00g of triphenylsulphonium bromide (prepared above) and 1.50g of sodium hexafluoroantimonate were mixed in 60 ml of acetone and stirred for 5 hrs. The suspension was filtered and the filtrate evaporated to yield a white solid in quantitative yield. Recrystallization from anhydrous ethanol gave white needles m.p. 178-9°C (88% recovery).
D. 0.5g of triphenylsulphonium bromide (prepared above) and 0.37g of sodium hexafluoroantimonate were mixed in 60 ml of ethylacetate and stirred for 5 hrs. The suspension was filtered and the filtrate evaporated to yield a white solid in quantitative yield. Recrystallization from anhydrous ethanol gave white needles m.p.

178-9°C (82% recovery).

E. 3.92g of tris(4-methylphenyl)sulphonium bromide (prepared as above) and 1.30g of sodium hexafluoroantimonate were mixed in 60 ml acetone and stirred 2 hrs. The suspension was filtered and the filtrate evaporated to give a white solid in quantitative yield. Recrystallization from anhydrous ethanol gave white needles m.p. 145-7°C (90% recovery).

F. 2.23g of tris(4-chlorophenyl)sulphonium bromide (prepared above) and 1.30g of sodium hexafluoroantimonate were mixed in 60 ml acetone and stirred for about 1 hr. The suspension was filtered and the filtrate evaporated to give a white solid in quantitiative yield. Recrystallization from anhydrous ethanol gave white needles m.p. 197-8°C (90% recovery).

## Claims

1. A process for the synthesis of a triarylsulphonium salt by the reaction of a aryl Grignard reagent with diarylsulfoxide followed by a metathetical reaction with a compound of the formula $ZMF_6$ where Z is a metal or metal-like, such as for example ammonium ion and M is antimony, arsenic or phosphorus, characterized in that the first step of the reaction is carried out in a solvent which is a mixture of aromatic and aliphatic hydrocarbons.

2. A process as claimed in claim 1 wherein the metathetical second step is carried out in a non-aqueous solvent.

3. A process as claimed in claim 1 wherein ammonium hexafluorophosphate is used in the metathetical reaction.

4. A process as claimed in claim 1 wherein ammonium hexafluoroantimonate is used in the metathetical reaction.

5. A process as claimed in claim 2 where ethylacetate is used as the solvent in the metathetical reaction.

6. A process as claimed in claim 2 wherein acetone is used as the solvent in the metathetical reaction.

7. A process as claimed in claim 2 wherein acetonitrile is used as the solvent in the metathetical reaction.

8. A process as claimed in claim 2 wherein ethanol is used as the solvent in the metathetical reaction.

9. A process as claimed in any preceeding claim wherein Z is the ammonium ion.

10. A process as claimed in any one of claims 1 to 8 wherein Z is sodium or potassium.

## Patentansprüche

1. Verfahren zur Synthese eines Triarylsulfoniumsalzes durch die Reaktion eines Aryl-Grignard-Reagens mit Diarylsulfoxid, gefolgt von einer Metathesereaktion mit einer Verbindung der Formel $ZMF_6$, bei welcher Z ein Metall oder Metallartiges, wie z.B. ein Ammoniumion, ist und M Antimon, Arsen oder Phosphor ist, dadurch gekennzeichnet, daß der erste Reaktionsschritt in einem Lösungsmittel durchgeführt wird, das eine Mischung aromatischer und aliphatischer Kohlenwasserstoffe ist.

2. Verfahren nach Anspruch 1, bei welchem der metathetische, zweite Schritt in einem nichtwäßrigen Lösungsmittel durchgeführt wird.

3. Verfahren nach Anspruch 1, bei welchem in der Metathesereaktion Ammoniumhexafluorphosphat verwendet wird.

4. Verfahren nach Anspruch 1, bei welchem in der Metathesereaktion Hexafluoroantimonat verwendet wird.

5. Verfahren nach Anspruch 2, bei welchem in der Metathesereaktion Essigsäureethylester als Lösungsmittel verwendet wird.

6. Verfahren nach Anspruch 2, bei welchem in der Metathesereaktion Aceton als Lösungsmittel verwendet wird.

7. Verfahren nach Anspruch 2, bei welchem in der Metathesereaktion Acetomitril als Lösungsmittel verwendet wird.

8. Verfahren nach Anspruch 2, bei welchem in der Metathesereaktion Ethanol als Lösungsmittel verwendet wird.

9. Verfahren nach irgendeinem der vorgehenden Ansprüche, bei welchem Z das Ammoniumion ist.

10. Verfahren nach irgendeinem der Ansprüche 1 bis 8, bei welchem Z Natrium oder Kalium ist.

## Revendications

1. Procédé pour la synthèse d'un sol de triarylsulfonium par la réaction d'un réactif aryle de Grignard avec un diarylsulfoxyde suivie d'une réaction métathétique avec un composé de formule $ZMF_6$ dans laquelle Z est un métal ou ressemble à un métal, comme par exemple, un ion ammonium et M est un atome d'antimoine, d'arsenic ou de phosphore, caractérisé en ce que la première étape de la réaction est effectuée dans un solvant qui est un mélange d'hydrocarbures aromatique et aliphatique.

2. Procédé suivant la revendication 1, dans lequel la seconde étape métathétique est effectuée dans un solvant non aqueux

3. Procédé suivant la revendication 1, dans lequel l'hexafluorophosphate d'ammonium est utilisé dans la réaction métathétique.

4. Procédé suivant la revendication 1, dans lequel l'hexafluoroantimoniate d'ammonium est utilisé dans la réaction métathétique.

5. Procédé suivant la revendication 2, dans lequel l'acétate d'éthyle est utilisé comme solvant dans la réaction métathétique.

6. Procédé suivant la revendication 2, dans lequel l'acétone est utilisée comme solvant dans ta réaction métathétique.

7. Procédé suivant la revendication 2, dans lequel l'acétonitrile est utilisé comme solvant dans la réaction métathétique.

8. Procédé suivant la revendication 2, dans lequel l'éthanol est utilisé comme solvant dans la réaction métathétique.

9. Procédé suivant l'une quelconque des revendications précédentes, dans lequel Z est l'ion ammonium.

10. Procédé suivant l'une quelconque des revendications 1 à 8, dans lequel Z est du sodium ou du potassium.